# EUROPEAN PATENT SPECIFICATION

(11) **EP 0 788 553 B1**
(45) Date of publication and mention of the grant of the patent: **03.04.2002**
(21) Application number: 94920556.1
(22) Date of filing: 13.07.1994
(51) Int. Cl.: C12Q 1/04, C12Q 1/06, C12Q 1/48, C12Q 1/66

(54) **MICROBIOLOGICAL TEST METHOD AND REAGENTS**
MIKROBIOLOGISCHES TESTVERFAHREN UND REAGENZIEN
PROCEDE D'ANALYSE MICROBIOLOGIQUE ET REACTIFS

(43) Date of publication of application: 13.08.1997
(73) Proprietor: The Secretary of St. for Def. in Her Britannic Maj. Governm of the Un. Kingdom of Gr. Brit. and N. Ireland, Salisbury, Wiltshire SP4 OJQ (GB)
(72) Inventor: SQUIRRELL, David, James, Wiltshire SP4 0JG (GB)
(74) Representative: Skelton, Stephen Richard
(86) International application number: GB9401513
(87) International publication number: WO9602665

(56) References cited:
- EP-A- 0 054 676
- WO-A-94/17202
- CH-A- 678 065
- US-A- 3 933 592
- H.U. BERGMEYER 'Methods of Enzymatic Analysis, Volume III, Third Edition' 1986 , VERLAG CHEMIE , WEINHEIM, DE see Chapter 7.5.3 :"Adenylate Kinase - Luminometric Method" by Sven E. Brolin see page 553 - page 559
- JOURNAL OF BIOCHEMICAL AND BIOPHYSICAL METHODS, vol.1, no.3, 1979, AMSTERDAM, NL pages 163 - 169 SVEN E. BROLIN ET AL. 'Photokinetic microassay of adenylate kinase using the firefly luciferase reaction' cited in the application
- Sigma Chemical Co., (1992) Catalog of commercial products (page 45)
- T 0802/92

## Description

The present invention relates to a method for detecting and assaying microorganisms, to agents for use in such a method, and to test kits comprising essential reagents for carrying out the method.

All living organisms utilise adenosine triphosphate (ATP) as a source of chemical energy and it is known to assay this using the ATP driven luciferase/luciferin reaction. Light generated by this enzymic reaction can be measured using a luminometer and related to the amount of ATP present. The usefulness of ATP as an index of microbial numbers has been known since the mid 1960's (see ATP Luminescence Rapid Methods in Microbiology (1989) editor Stanley et al.; Blackwell Scientific Publications, London, see pages 1-10); its main advantage being speed and sensitivity. Utilising this assay format simple samples can be analysed in a matter of minutes while complex ones routinely take only half an hour with a detection capability provided down to 10⁻¹² mol/l ATP. There is however a need for methods which provide still further sensitivity when detecting microorganisms or their contents while retaining speed and ease of performance.

The present inventor has now determined that the speed and sensitivity of ATP based method can be enhanced significantly by shifting the target of the assay from ATP to an enzyme which generates it, particularly to adenylate kinase. Adenylate kinase is an enzyme used by all organisms for the conversion of adenosine diphosphate (ADP) to adenosine triphosphate (ATP). The targeting of this enzyme in preference to ATP, by using the preferred method, reagents and kits of the invention, allows the detection of down to at least 10⁻²⁰ moles of intracellular marker adenylate kinase.

It is known to assay adenylate kinase using the luciferase/luciferin system (see Brolin et al Journal of Biochemical and Biophysical Methods 1 (1979) 163-169 and Shutenko et al. Biotekhnologiya, No 4. (1988) 542-547) for the purpose of determining its activity and this has been applied to study of certain mammalian and plant tissues (eg. see Rodionova et al Fiziologiya Rastenii (1978) 25, 4, P731-734). The use of such assay system for the detection and assay of microorganisms however has not been suggested and the advantages of doing such, ie. enhanced sensitivity so provided, have not been relevant to those studying the enzyme itself.

Although adenylate kinase is present in smaller quantities than ADP or ATP, its use as a biological marker for microorganisms provides enhanced sensitivity with a typical amplification available of 400,000 by measuring its presence through the ATP it produces; that is for every mole of enzyme present 400,000 moles of ADP are converted to ATP in a 10 minute incubation. Thus estimation of the enzyme by measuring the substrate or product of the reaction it catalyses provides for detection down to as low as 10⁻²⁰ moles.

The applicant's copending PCT application WO 94/17202 relates to the general method of estimation of microorganisms in a sample from the sample's ability to convert ADP to ATP, and relating that to the presence of microorganisms or their intracellular materials. That application exemplifies the method wherein magnesium ions, necessary for the reaction of two molecules of ADP with each adenylate kinase active site, are not added as a reagent but are provided by any bacteria cells present and as an impurity in the other reagents. The number of cells detected in the examples using such technique proved to be about 10², with results of a more statistically valid nature being obtained at 10³ or more; a linear relationship between luminometry counts and cell numbers then being obtainable.

The present invention relates to an improved technique in which adenylate kinase activity is measured in an optimised fashion using reaction conditions in which magnesium ions are supplied to the ADP conversion reaction, and wherein the reagents used are treated to remove adenylate kinase to higher degrees of purity whereby the number of microorganisms that can be detected is of the order of tens rather than hundreds per 200µl sample, and readings with linear relation between cells and ATP derived light are possible down to 10 cells.

In a first aspect of the present invention there is provided a method for determining the presence and/or amount of microorganisms and/or their intracellular material present in the sample characterised in that the amount of adenylate kinase in the sample is estimated by mixing it with adenosine diphosphate (ADP) and a source of magnesium ions, added as a reagent, determining the amount of adenosine triphosphate (ATP) produced by the sample from this ADP, and relating the amount of ATP so produced to the presence/or amount of adenylate kinase and to microorganisms and/or their intracellular material. Magnesium ions are suitably added at a molar concentration sufficient to allow maximal conversion of ADP to ATP. The amount of magnesium added is preferably such that there is sufficient to provide one mole of magnesium ions for one mole of ADP such that all of the ADP molecules may be associated with at least one magnesium ion.

In preferred embodiments of this aspect of the invention the sample is provided in the form of an aqueous suspension or solution and the estimation of adenylate kinase, and thus microorganisms and/or intracellular material in the sample, is carried out by adding ADP and magnesium ions to the sample under conditions whereby any adenylate kinase present will convert ADP to ATP, incubating the sample for a predetermined period to effect such conversion, adding luciferase and luciferin agents, determining the amount of light emitted from the sample and relating that to presence and amount of adenylate kinase.

The amount of ADP with which the sample is mixed is preferably sufficient to provide an ADP concentration in the mixture in excess of 0.005mM, more preferably in excess of 0.01mM and most preferably in excess of 0.08mM. A particularly preferred amount of ADP in the conversion step mixture is about 0.1mM.

Where reagents are to be used which contain magnesium ion depleting agents, eg. chelating/sequestering agents such as EDTA and phosphate buffers, it will be realised that in order to provide the ADP with sufficient magnesium ions for it to undergo optimal conversion, it will be preferred for an excess of magnesium ions to be present. For the preferred concentrations of ADP set out above, the preferred concentration of magnesium ions in the suspension or solution during conversion of ADP to ATP is 1mM or more, more preferably 5mM or more and most preferably 10mM or more. The magnesium ions may be provided in the form of any magnesium salt, preferably as magnesium acetate.

A further preferred format of the present invention adds luciferin /luciferase luminometry reagents to the sample at the beginning of the incubation, preferably as a single reagent with the ADP and magnesium ion source. Luciferase is preferably stored separately from extractant.

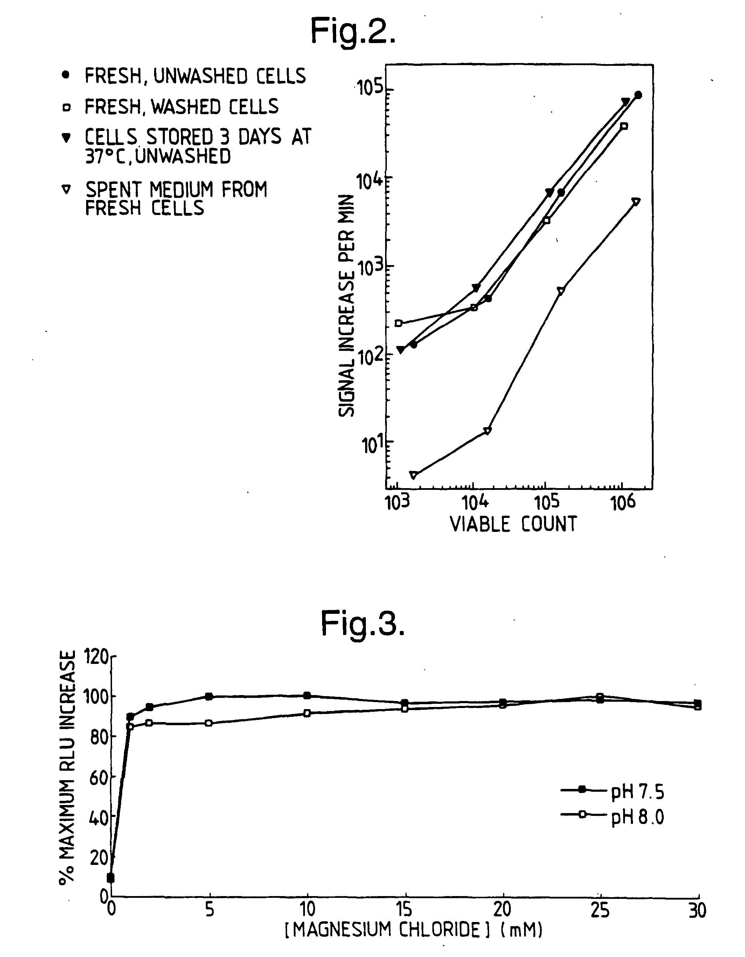

In formats of the invention where all the reagents are included at the start of the conversion of ADP to ATP in this manner, and/or where luminometer counting is continued after luciferin/luciferase addition where that is a separate step, magnesium may be provided by the luciferin/luciferase reagent. However, due to binding of magnesium ions by EDTA and phosphate it is necessary that the amount of magnesium ions is positively ensured by prior experiment or calculation. It will be realised by those skilled in the art that the optimal amount of magnesium salt to be added to a given ADP, sample and luciferin /luciferase mixture will be readily determinable by routine experiment using a sample containing a known amount of bacteria, eg. E. coli. whereby maximal signals are obtained. Figure 3 below gives an indication of the optimal amount of magnesium acetate to be added to a mixture as used in the Examples below.

As Mg²⁺ ions facilitate ADP depletion due to contaminant adenylate kinase, it is preferred not to keep them in solution together prior to use; chelating agent such as EDTA may be included in the ADP to prevent this. Preferably the magnesium and ADP are brought together just prior to use or in the ADP conversion step. Where the reagents are to be kept together it is preferred that they are kept in freeze dried form to avoid any premature ADP conversion to ATP.

As stated above, although other assays may be used. ATP is preferably detected by use of the luciferin/luciferase system to provide a photometrically detectable signal indicative of the amount of ATP in the sample. Luciferin/luciferase preparations and methods for their use in assaying ATP will be well known to those skilled in the art and are commercially available (eg. see Brolin et al). A typical formulation contains eg. 0.1 to 10mg/litre luciferase, 15 to 1000µmol/litre D-luciferin, and agents such as MgCl₂ (2.5-25mmole), EDTA, BSA, and pH7 buffer (see eg. EP 054676).

For single reagent use with adenylate kinase testing methods as described herein it is preferred that the pH is adjusted to that which is optimal for both enzymes, ie. a compromise, in order that counting might continue while converting ADP to ATP. This may be determined by routine experiment using known bacterial numbers in a sample.

The sample. ADP and magnesium ion source may be mixed in any buffer providing a pH suitable for the adenylate kinase reaction; no other reagents are necessary. Thus any buffer providing a pH of between 5.5 and 8.5 might be used, with optimal pH lying between pH6 and 7, preferably pH6.5. Examples of suitable buffers include Tris and phosphate buffers. Most suitably the sample is collected and/or diluted in such a buffer in preparation for carrying out the method of the invention.

As with any amplified assay, the sensitivity of the adenylate kinase assay of the present invention is limited by the purity of the reagents. In this case the significant contaminants are ATP in the ADP substrate and adenylate kinase in the luciferase preparation. For use as a sensitive assay for microorganisms, particularly where these may be potentially harmful and need detecting in low numbers, it is necessary that the purity of each of the reagents be as high as possible with respect to the substance with which it is to react in the assay.

To address the first problem, high purity commercial ADP (>99.5% purity) is preferably used after further purification by column chromatography. This is desirable because even small amounts of contaminating ATP may be sufficient to cause a high background reading. For example, using a diethylaminoethylcellulose column and 0.02mM hydrochloric acid eluent, ATP is eluted more slowly from the column than ADP to a degree enabling substantial separation. Other chromatographic media and eluent combinations may also be used to similar effect, for example HPLC using Nucleosil (Registered Trademark-RTm) column packings (available from Technicol, Stockport Cheshire UK), such as Nucleosil 3 (RTm) and Nucleosil 5 (RTm),, using 0.06M KH₂PO₄:methanol in 77:23 ratio v/v at pH6 with 5mM tetrabutylammonium hydrogen sulphate. Fractions with high ADP to ATP ratios are retained for use and purity is assessed by luciferin/luciferase reagent mediated bioluminescence after adenylate kinase action to measure ADP levels and without adenylate kinase to measure ATP contaminant levels.

Using a preferred Econopaq Q (RTm) strong anion exchange gel cartridge (Biorad-RTm) equilibrated with 20mM potassium phosphate at pH4.6 and eluting with steps of KPᵢ concentration up to 400mM, ADP was found to be strongly retained and eluted as a coherent peak, with ATP eluting after it. In this manner ADP with a molar % ATP upper limit of 2 x 10⁻⁸ was obtainable. The most pure ADP the applicants are aware of from the literature is 0.001% (see Shutenko et al, as above) thus the present invention provides ADP for use in the method of the present invention that has less than 0.001 molar % ATP, more preferably 2 x 10⁻⁸ molar % or less.

A further method for removing ATP from the ADP substrate uses enzymes that specifically degrade ATP, such as luciferase or apyrase. Such enzymes may also be used to further purify chromatographically purified ADP, or alternatively enzymically purified ADP may be treated by column chromatography. It will be noted that apyrase is also an ADPase, but as some are more active on ATP and the ADP is present at much higher levels this does not present a significant problem.

With regard to the second problem, adenylate kinase, as an essential "housekeeping" enzyme, is present in virtually all organisms and is generally present in luciferase preparations. It may only be a minor contaminant, but since the aim is to measure very low adenylate kinase levels in samples. its presence in the luciferase may be a limiting factor. In fact the applicant has determined that, defining one unit (U) of activity as the amount of enzyme which converts 1µmol ADP to 1µmol ATP per minute in the presence of 0.5mM ADP and 4.5mM Mg²⁺ at pH7.8 at 20°C, commercial luciferases may contain 10⁻⁷U/ml or more adenylate kinase activity whereas luciferin, its substrate, comprises very little if any activity. Furthermore it is common to stabilise luciferase reagents with a stabiliser, commonly a protein such as bovine serum albumin (BSA), and commercial preparations of this have been determined by the applicant to possess significant adenylate kinase activity.

The molecular weights of luciferase and adenylate kinase are significantly different, being 61kD and 21kD respectively. Furthermore luciferase is a membrane bound protein and therefore relatively hydrophobic, whereas adenylate kinase occurs as a soluble enzyme. It is thus possible to remove adenylate kinase from luciferase preparations by, eg. size exclusion chromatography, reverse phase chromatography, or both. Alternatively or in addition to this, the problem of adenylate kinase contamination of luciferase can be avoided by adding the bioluminescent reagents (luciferase and luciferin) just before or as measurements are taken so that any contaminating adenylate kinase does not have the time to produce a significant effect.

Suitable methods for purifying luciferase use column chromatography fractionation with a low porosity gel, eg Sephadex G-25 (RTm) (see Nielsen and Rasmussen, Acta Chemica Scandinavica 22 (1968) p1757-1762; use of Sephadex (RTm) and Sepharose (RTm) columns (eg Blue Sepharose) in series and/or SDS electrophoresis (see Devine et al, Biochimica et Biophysica Acta 1172 (1993) 121-132) or ageing for a period at elevated ambient temperature.

In order to remove adenylate kinase activity from agents such as bovine serum albumin it is similarly possible to use column chromatography. A further treatment that has proved successful in this regard is chemical treatment of the BSA such that its ability to stabilise the luciferase is retained, but adenylate kinase activity is reduced or depleted altogether. Any conventional chemical treatment for the depletion of enzymic activity from proteins may be equally be applied for this purpose. Alternatively a non-protein luciferase stabiliser, eg. glycerol, may be used as a supplement or replacement for the BSA.

For example, the applicant has determined that commercially available BSA can have its adenylate kinase activity reduced to less than 2% of its original activity or less merely by heat treatment at acid or alkaline pH. One suitably effective treatment heats the BSA at pH5.6 or pH10 at 50°C for 24 hours. A further source of adenylate kinase free BSA is the chemically treated reagent acetylated-BSA, available from Sigma and BDH. It will be realised by those skilled in the art that other chemically treated BSAs will also be suitable.

In order to render all the adenylate kinase associated with a target microorganism available to the ADP, magnesium ions and luciferase /luciferin assay reagents of the invention it will be necessary to disrupt them such that intracellular material is released or otherwise exposed to the reagents. Such disruption might be carried out using mechanical means such as an ultrasonic generator, by use of osmotic shock optionally in association with cold shock or such agents as lysozyme or, more conveniently, by use of detergents. Such detergents are commercially available and commonly referred to as 'extractants'. Typical extractants include generic cationic detergents such as CTAB (cetyl trimethyl ammonium bromide), and proprietory agents such as Biotrace (RTm) XM extractant (available from Biotrace. Bridgend UK), Celcis UK cationic extractants and Lumac NRM (RTm) (nucleotide releasing agent available from Lumac BV, Holland). When using CTAB a convenient preparation will include 0.01 to 1% CTAB in water. eg 0.2%, but other concentrations may occur to those skilled in the art.

Thus before adding ADP and luciferase/luciferin reagent(s) to an assay sample suspected of containing microorganisms it is preferred to disrupt these to render their intracellular contents accessible to luminometry reagents by use of disrupting agent. If it is desired to. distinguish between target cells and cells such as those of fungal spores it is possible to run two separate assays treating one with a nonionic detergent capable of disrupting only these spores and multi -cellular 'somatic' animal cells (eg.Triton TX-100-RTm) and the other with cationic detergent 'extractants' detailed above for disrupting all cells. It is possible to carry out these assays on the same sample if an ATPase such as apyrase is added between detergent/luciferase /measurement cycles; one cycle using nonionic and the other cationic detergent in a first cycle step with filtration steps between.

The effect of extractant upon the luciferase/luciferin system is known to be important (see eg. Simpson et al (1991) J. Biolumin Chemilumin 6(2) pp97-106); with cationic detergents being known to potentiate the reaction but to cause gradual inactivation of luciferase, anionic detergent inhibiting the reaction and nonionic and zwitterionic detergents being known to potentiate over a wide range. A mixture of 0.15% cationic detergent together with 0.25% tertiary diamine surfactant (obtained from Celcis, Cambridge, UK) was found to be satisfactory for present purposes, but those skilled in the art will have no problem screening for other 'extractants' that yield an optimal mix of adenylate kinase and luciferase activity when copresent in the same solution.

The light given off from the mixture after all the essential steps are complete, ie. ADP conversion to ATP and subsequent action of luciferase upon luciferin, may be measured by residence of the sample volume, eg. luminometer tube, within a light detector immediately after or simultaneously with addition of the luciferase and luciferin or other agents which enable the essential steps to proceed.

In a second aspect of the present invention there is provided a test kit comprising the essential reagents required for the method of the invention, ie. adenosine diphosphate, a source of magnesium ions and preferably luciferase and luciferin. Preferably the kit includes all these reagents, with the luciferase and luciferin being provided as a single reagent solution, with a detergent reagent in the kit suitable for disrupting the target cells for which the assay is intended. Usually for assaying microorganisms only cationic detergent is needed, whereas if fungal spores and somatic cells are likely to be significant then a further nonionic detergent reagent might be included to assess their numbers. The kit is in the form of a single package preferably including instructions as to how to perform the method of the invention; the reagents being provided in containers and being of strength suitable for direct use or after dilution.

It'may be appropriate to provide the magnesium ions with the luciferase/luciferin reagent if this is to be added before the ADP to ATP conversion has begun, but then they should be in excess over that bound to the EDTA or phosphate in that reagent and should be optimised to accomodate both adenylate'kinase and luciferase requirements. For microbial detection magnesium ions are preferably provided with a sample collection/dilution buffer but other formats may be preferred for particular applications. Most conveniently the magnesium ions are provided with a sample collection or dilution buffer, the ADP is provided with detergent/surfactant extractants and optionally with a stabiliser such as EDTA, and the luciferase and luciferin are provided together, thus providing a three reagent test kit. Alternatively these agents may be provided in the form of a single reagent that is freeze dried such that they do not interact to cause degradation of eg. the ADP, prior to use.

A preferred test kit of the invention comprises ADP reagent which is of purity higher than 99.999%, and a luciferase/luciferin reagent, including BSA, that is substantially free of adenylate kinase activity. Alternatively the luciferase/luciferin ratio used, reflected in the kit instructions for use and/or in their relative concentrations, is such that the luciferase is capable of acting upon the luciferin substrate sufficiently quickly such that any luciferase associated adenylate kinase produces ATP after the initial emission is finished; thus microorganism derived adenylate kinase will be indicated by a flash kinetic reaction and contaminant ATP by a glow.

The preferred purified reagents may be provided by the methods described above. It is noted that adenylate kinase activity in luciferase may also be depleted by leaving the luciferase to stand for a period of months or years.

The methods, apparatus, reagents and kits of the present invention will now be illustrated by way of example only with reference to the following non-limiting Examples and Figures. Further embodiments of the invention will occur to those skilled in the art in the light of these.

### FIGURES

Figure 1: plots log luminometer signal against log number of E. coli in a 200 µl sample using the improved assay of the invention using 1 and 5 minute incubations prior to luciferin/luciferase addition.
Figure 2: plots log luminometer signal against log number of cells of E. coli in absence of magnesium.
Figure 3: shows the effect of magnesium ion concentration upon the luminometer signal derived from a set number of P. aeruginosa at pH 7.5 and pH 8.0 showing the 10 fold increase over no added magnesium.

### Example 1: Preparation of purified adenosine diphosphate reagent.

Liquid chromatography was used to further purify commercial high purity (>99.95%) ADP (Sigma) using a 5ml Econopac Q cartridge (RTm) (Biorad-RTm) equilibrated with 20mM potassium phosphate pH4.6 and loaded with 5ml of the 1mM ADP (2.1 mg). Elution was carried out by steps of KPᵢ concentration up to 400mM whereupon the ADP was strongly retained and eluted as a peak at approximately 340mM KPᵢ. Setting the system up with a pump (5ml/min) and gradient mixer, a gradient of 50 to 1M KPᵢ in 200ml total was provided and 5ml fractions collected. ADP eluted as a sharp peak between fractions 12 and 17 with ATP beginning to appear at the end of the gradient. A step in [KPᵢ] to 1 M eluted the remaining ATP. The purest ADP fractions from this column were of less than 2x 10⁻⁸ mole % ATP.

### Example 2: Preparation of adenylate kinase free luciferase reagents.

Adenylate kinase activity was deleted from commercially available luciferin/luciferase reagents (Biotrace HM RTm) by ageing, including several months at high ambient temperature (circa 30°C) over a period of 12 months in dry form.

### Example 3: Alternative preparation of kinase free luciferase reagents.

Commercially available luciferase is purified using column chromatography by the method of Devine et al (1993) using Blue Sepharose (RTm) as referred to above.

### Example 4: Preparation of adenylate kinase free BSA.

Sigma Fraction V (RIA Grade, Cat. No. A-7888) BSA was made up at 1% weight/volume in 200ml sterile water to give a starting pH of 5.6. Two 50ml samples of this were put into 100ml Duran bottles, the remainder made to pH10 using 5M NaOH and 50ml put into each of two Durans. Thimerosal was added to 0.02% final concentration as a preservative to prevent microbial growth and the bottles incubated at 37°C or 50°C for 24 hours before the pH of each was readjusted to 7.6 with 5M HCl or 5M NaOH as appropriate. Adenylate kinase activity was measured by mixing 100µl BSA sample as prepared above with 100µl 30mM magnesium acetate solution, placing the resultant mixture in a 3.5ml luminometer tube in a luminometer and adding 100µl ADP solution prepared in Example 1 and 100µl luciferin /luciferase reagent (Celcis, Cambridge UK) that was prepared free of adenylate kinase activity by use of column chromatography and chemically treated BSA. After a 5 second delay light emission integrated over 10 seconds was measured and recorded on a computer and a total of 10 sequential 10 second readings were made to determine the rate of ATP production; analyses being performed in duplicate. Calibration was made using 4 replicate measurements of the light emitted from 5µl of 10ng/ml (91 femtomoles) of ATP in water: mean signal 2950 per femtomole.

Results: The BSA sample incubated at 37°C remained clear whilst those at 50°C formed a precipitate which was slight at pH10 and very heavy at pH5.6. At pH10 and 50°C there was slight discolouration. Adenylate kinase activity remaining in these samples is shown below in Table 1 below as represented by luminometer counts per minute.

It is recommended that still milder forms of inactivation are used, with longer duration, or that the BSA is immediately freeze dried, if it is intended to store it for any length of time as after 2 weeks even the pH10 50°C sample became unuseable due to increased discolouration. The 37°C samples did not go off in this manner and thus offer better scope for reducing stable adenylate kinase free BSA by increasing the incubation time. The fact that the Biotrace HM (RTm) agent lost its activity in dry form after storage at 40°C demonstrates the possibilities here.

**TABLE 1:**

| Treatment | Counts t5-15 | t95-105 | Difference | d[ATP]/dt (fm.sec⁻¹) |
|---|---|---|---|---|
| 37/5.6 | 9350 | 41727 | 23207 | 7.1 |
| | 9845 | 26041 | (means) | |
| | 11896 | 32945 | | |
| 37/10 | 7192 | 30602 | 17943 | 5.5 |
| | 5047 | 20557 | | |
| | 4469 | 19377 | | |
| 50/5.6 | 606 | 1191 | 595 | 0.18 |
| | 343 | 948 | | |
| 50/10 | 460 | 1014 | 5.00 | 0.15 |
| | 342 | 847 | | |
| | 314 | 754 | | |

### Example 5: Preparation of luciferin/luciferase reagent with BSA.

Commercial preparations of luciferin/luciferase commonly contain BSA as necessary. BSA chemically treated as set out in Example 4 above or as commercially available as acetylated BSA (eg BDH or Sigma) was admixed with adenylate kinase free luciferase in normal proportions together with other standard Celcis agents such as to provide a Celcis LDR luciferin/luciferase luminescence reagent of adenylate kinase activity less than 10⁻⁹U assay volume (ie. 300µl).

### Example 6: Test kit of the invention.

A test kit of the invention is provided consisting of the following:
(i) a container holding 15mM magnesium acetate solution for collection /dilution of samples;
(ii) a container holding purified ADP solution (>99.99999998% pure with regard to ATP) prepared as described in Example 1 in a concentration of 0.3mM in potassium phosphate (7.5mM pH6.5) buffer solution further including 0.2mM EDTA and a mixed extractant of 0.15% cationic detergent and 0.25% tertiary diamine surfactant.
(iii) a container holding luciferin/luciferase LDR (Celcis, Cambridge, UK) bioluminescence reagent of adenylate kinase activity less than 10⁻⁸U/100µl.

Optionally included in the package is a container of nonionic detergent solution (Triton X-100 (RTm) 0.2% or equivalent) and/or a container holding an ATPase such as apyrase for the destruction of ATP released by the action of the nonionic detergent on a sample rendering it suitable for reassay by addition of the cationic detergent.

### Example 7: Assay of known amounts of E.coli using method of the invention.

A one week old E. coli broth culture containing approximately 2.2 x 10⁷ microorganisms per 200µl of phosphate buffered saline pH7.4 was used as stock and diluted in successive dilutions of 10 with the collection/dilution reagent containing magnesium ions ((i) in Example 6) to give a range of samples of from 10⁷ to 0.1 microorganisms per 200µl sample.

Each 200µl sample was added to a 3.5ml luminometer tube, 100µl of ADP/extractant reagent ((ii) in Example 6) added and the mixture, total volume 300µl, was incubated at room temperature for 1 or 5 minutes. On completion of the incubation 100µl of modified Celcis LDR bioluminescence reagent, ((iii in Example 6 above) was added and the light emitted determined over a first 10 second interval and then over 10 second intervals up to one minute to determine the increase in light in cumulative fashion using a Biotrace M3 (RTm) luminometer. The initial signal value was subtracted from the final reading to gain a measure of the signal in counts per minute.

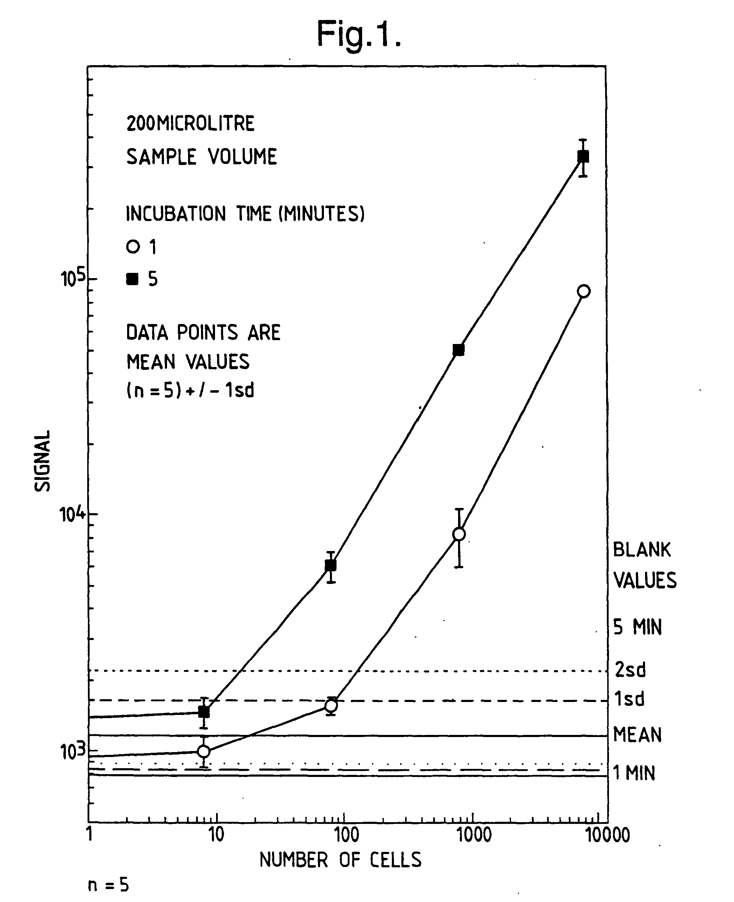

The efficacy of the present method can be seen by reference to Figure 1 where statistically valid linear response between number of E.coli and light emitted by the sample mix after 5 minute incubation with ADP is obtained for 10 organisms per sample and upward, for 100 organisms and upward with a 1 minute incubation, and a detection limit of about 10 organisms is given in both cases. This compares very favourably with the method of WO 94/17202 which gives a difference of only 26 cpm after a 1 minute incubation with 100 organisms and 67 cpm with 1000; linear responses only being obtained with 1000 cells and over. By comparison 1000 cells per sample in the present method gives a signal increase of several thousand cpm after 1 minute.

It will be realised that in order to perform an assay for an unknown number of microorganisms in a sample using the present method, a calibration curve may be provided plotting known numbers of microorganisms against luminometer counts as shown in the Figures 1 and 2 (eg. as log values), deriving a number of counts from a sample containing the unknown number of microorganisms (including zero organisms) using the same protocol, and estimating the number of microorganisms in the sample as being that corresponding to the same number of counts on the curve.

It will be realised by those skilled in the art that the amount of adenylate kinase present in a particular microorganisms, eg.bacteria, may vary from other microorganisms. For example, yeasts contain more adenylate kinase than bacteria by virtue of their size, and indeed single yeasts can be detected by this method. Thus for a given microorganism a particular calibration curve may be required, and it may be necessary to provide such curves for different states of the same microorganism, eg. for weakened, pH or oxygen stressed organisms. However, a further advantage of the present method over existing ATP based methodology is that adenylate kinase content will be more closely correlated to cell numbers than the highly variable ATP content which is depleted by cell metabolism.

## Claims

1. Method for determining the presence and/or amount of microorganisms and/or their intracellular material present in a sample **characterised in that** the amount of adenylate kinase in the sample is estimated by mixing the sample with adenosine diphosphate (ADP) and a source of magnesium ions added as a reagent, determining the amount of adenosine triphosphate (ATP) produced by the sample from this ADP, and relating the amount of ATP so produced to the present/or amount of microorganisms and/or their intracellular material.

2. A method as claimed in claim 1 wherein the magnesium ions are added at a molar concentration sufficient to allow maximal conversion of ADP to ATP.

3. A method as claimed in claim 1 or claim 2 wherein the amount of magnesium ions added is such that there are sufficient magnesium ions present to provide one mole of magnesium for one mole of ADP such that all of the ADP molecules may be associated with at least one magnesium ion.

4. A method as claimed in any one of claims 1 to 3 wherein the sample is provided in the form of an aqueous suspension or solution and the estimation of microorganisms and/or intracellular material therein is carried out by adding ADP and magnesium ions to the sample under conditions whereby any adenylate kinase present will convert ADP to ATP, incubating the sample for a predetermined period to effect such conversion, adding luciferase and luciferin agents, determining the amount of light emitted from the sample and relating that to presence and amount of microorganism and/or intracellular material.

5. A method as claimed in any one of claims 1 to 4 wherein the amount of ADP with which the sample is mixed is sufficient to provide an ADP concentration in the mixture in excess of 0.005mM.

6. A method as claimed in claim 5 wherein the ADP is in excess of 0.08mM.

7. A method as claimed in claim 5 wherein the ADP concentration is about 0.1mM.

8. A method as claimed in any one of the preceding claims wherein the concentration of magnesium ions in the suspension or solution during conversion of ADP to ATP is 1mM or more.

9. A method as claimed in claim 8 wherein the concentration of magnesium ions in the suspension or solution is 10mM or more.

10. A method as claimed in any one of the preceding claims wherein the magnesium ions are provided in the form of magnesium acetate.

11. A method as claimed in any one of the preceding claims wherein the luciferin/luciferase luminescence reagents are added to the sample at the beginning of the incubation as a single reagent with the ADP and magnesium ion source.

12. A method as claimed in any one of the preceding claims wherein the magnesium ion source and the ADP are kept in dry form or in separate solutions prior to use and brought together or made into an aqueous solution immediately prior to use or in the ADP conversion step.

13. A method as claimed in any one of the preceding claims wherein the magnesium ion source and sample are mixed together before adding the ADP.

14. A method as claimed in claim 13 wherein the sample is collected or diluted in a solution comprising the magnesium ion source.

15. A method as claimed in any one of the preceding claims wherein the conversion of ADP to ATP is carried out at a pH of between 5.5 and 8.5.

16. A method as claimed in any one of the preceding claims wherein the ADP has a molar % ATP of less than 0.001%.

17. A method as claimed in claim 16 wherein the ADP has a molar % ATP of 2 x 10⁻⁸ or less.

18. A method as claimed in claim 16 wherein the ADP is stored in the presence of a chelating agent to prevent contaminant adenylate kinase converting it to ATP prematurely.

19. A method as claimed in any one of the preceding claims wherein the luciferase/luciferin reagent has an adenylate kinase content of less than 10⁻⁷U/ml.

20. A method as claimed in claim 19 wherein the luciferase/luciferin reagent comprises bovine serum albumin that has been chemically treated to deplete its adenylate kinase activity.

21. A method as claimed in any one of the preceding claims wherein the sample is. treated with an extractant which disrupts microorganism cells and exposes their adenylate kinase to the ADP and magnesium ions.

22. A method as claimed in claim 21 wherein the cells are fungal spores or somatic cells and the extractant comprises a non-ionic detergent.

23. A method as claimed in claim 21 wherein all cells are to be detected and/or quantified and the extractant comprises a cationic detergent.

24. A method as claimed in claim 23 wherein the extractant further comprises a surfactant.

25. A method as claimed in claim 21 wherein the cells are bacterial cells wherein the ATP released by non-ionic detergent is subtracted from the ATP released by cationic detergent and surfactant, and the remainder related to bacterial cell numbers.

26. A test kit for detection and/or quantification of microorganisms and/or their intracellular material by a method according to any one of claims 1 to 25; said kit comprising ADP and a source of magnesium ions together with an extractant for exposing microorganism adenylate kinase to these such that conversion of the ADP to ATP takes place.

27. A test kit as claimed in claim 26 further comprising luciferase and luciferin in the form of a bioluminescence reagent capable of emitting light in the presence of ATP.

28. A test kit as claimed in claim 27 wherein the source of magnesium ions is provided as a sample collection or dilution solution.

29. A test kit as claimed in claim 28 wherein the collection or dilution buffer comprises magnesium acetate.

30. A test kit as claimed in any one of claims 27 to 29 wherein the ADP is together with detergent and/or surfactant extractant.

31. A test kit as claimed in any one of claims 27 to 30 wherein the ADP, the magnesium ion source and the bioluminescence agent are provided in three separate containers.

32. A test kit as claimed in any one of claims 27 to 30 wherein the agents are all provided as a single freeze dried reagent.

33. A test kit as claimed in any one of claims 26 to 32 wherein the ADP is of purity higher than 99.999 mole % with respect to ATP.

34. A test kit as claimed in any one of claims 26 to 33 comprising a bioluminescence reagent of adenylate kinase activity less than 10⁻ ⁷U/ml.

35. A test kit as claimed in claim 33 wherein the bioluminescence reagent comprises bovine serum albumin that has been chemically treated to deplete its adenylate kinase activity.

36. A reagent comprising ADP of purity with respect to ATP of greater than 99.999%.

37. A reagent as claimed in claim 36 further comprising a chelating agent in sufficient amount to prevent contaminating adenylate kinase from converting ADP to ATP.

38. A reagent as claimed in claim 37 wherein the chelating agent comprises EDTA.

## Patentansprüche

1. Verfahren zur Bestimmung des Vorliegens und/oder der Menge von Mikroorganismen und/oder ihres intrazellulären Materials, die (das) in einer Probe vorliegen (vorliegt), **dadurch gekennzeichnet, daß** die Menge an Adenylatkinase in der Probe bestimmt wird, indem die Probe mit Adenosindiphosphat (ADP) und einer Magnesium-Ionenquelle, die als Reagens zugesetzt wird, vermischt wird, die Menge an Adenosintriphosphat (ATP), die durch die Probe aus diesem ADP produziert wird, bestimmt wird und die so produzierte Menge an ATP zum Vorliegen und/oder der Menge an Mikroorganismen und/oder ihrem intrazellulärem Material in Beziehung gesetzt wird.

2. Verfahren nach Anspruch 1, wobei die Magnesium-Ionen in einer molaren Konzentration zugesetzt werden, die ausreicht, um eine maximale Umwandlung von ADP in ATP zu ermöglichen.

3. Verfahren nach Anspruch 1 oder Anspruch 2, wobei die zugesetzte Menge an Magnesium-Ionen so ist, daß ausreichend Magnesium-Ionen vorliegen, um 1 mol Magnesium pro 1 mol ADP bereitzustellen, so daß alle ADP-Molekül mit mindestens einem Magnesium assoziiert werden können.

4. Verfahren nach einem der Ansprüche 1 bis 3, wobei die Probe in Form einer wäßrigen Suspension oder Lösung bereitgestellt wird und die Bestimmung von Mikroorganismen und/oder intrazellulärem Material darin durchgeführt wird, indem ADP und Magnesium-Ionen unter Bedingungen, bei denen eine vorhandene Adenylatkinase ADP in ATP umwandeln wird, zu der Probe gegeben werden, die Probe für einen vorbestimmten Zeitraum zur Durchführung einer solchen Umwandlung inkubiert wird, Luciferase- und Luciferin-Reagenz zugegeben wird, die Lichtmenge, die von der Probe emittiert wird, bestimmt wird und diese zum Vorliegen und der Menge von Mikroorganismus und/oder intrazellulärem Material in Beziehung gesetzt wird.

5. Verfahren nach einem der Ansprüche 1 bis 4, wobei die ADP-Menge, mit der die Probe vermischt wird, ausreichend ist, um eine ADP-Konzentration von über 0,005 mM in dem Gemisch bereitzustellen.

6. Verfahren nach Anspruch 5, wobei das ADP über 0,08 mM ausmacht.

7. Verfahren nach Anspruch 5, wobei die ADP-Konzentration etwa 0,1 mM beträgt.

8. Verfahren nach einem der vorangehenden Ansprüche, wobei die Magnesium-Ionenkonzentration in der Suspension oder Lösung während der Umwandlung von ADP in ATP 1 mM oder mehr beträgt.

9. Verfahren nach Anspruch 8, wobei die Magnesium-Ionenkonzentration in der Suspension oder Lösung 10 mM oder mehr beträgt.

10. Verfahren nach einem der vorangehenden Ansprüche, wobei die Magnesium-Ionen in Form von Magnesiumacetat bereitgestellt werden.

11. Verfahren nach einem der vorangehenden Ansprüche, wobei die Luciferin/Luciferase-Lumineszenzreagenzien zu Beginn der Inkubation als einzelnes Reagens mit dem ADP und der Magnesium-Ionenquelle zu der Probe gegeben werden.

12. Verfahren nach einem der vorangehenden Ansprüche, wobei die Magnesium-Ionenquelle und das ADP vor einer Verwendung in trockener Form oder in getrennten Lösungen gehalten werden und unmittelbar vor einer Verwendung oder im ADP-Umwandlungsschritt zusammengebracht oder in wäßrige Lösung gebracht werden.

13. Verfahren nach einem der vorangehenden Ansprüche, wobei die Magnesium-Ionenquelle und die Probe vor Zugeben des ADP miteinander vermischt werden.

14. Verfahren nach Anspruch 13, wobei die Probe in einer Lösung, die die Magnesium-Ionenquelle umfaßt, gesammelt oder verdünnt wird.

15. Verfahren nach einem der vorangehenden Ansprüche, wobei die Umwandlung von ADP in ATP bei einem pH zwischen 5,5 und 8,5 durchgeführt wird.

16. Verfahren nach einem der vorangehenden Ansprüche, wobei das ADP einen mol-%-Gehalt an ATP von weniger als 0,001 % hat.

17. Verfahren nach Anspruch 16, wobei das ADP einen mol-%-Gehalt an ATP von 2 x 10⁻⁸ oder weniger hat.

18. Verfahren nach Anspruch 16, wobei das ADP in Gegenwart eines Chelatbildners aufbewahrt wird, um zu verhindern, daß eine Adenylatkinase-Verunreinigung es vorzeitig in ATP umwandelt.

19. Verfahren nach einem der vorangehenden Ansprüche, wobei das Luciferase-/Luciferin-Reagens einen Adenylatkinase-Gehalt von weniger als 10⁻⁷ E/ml hat.

20. Verfahren nach Anspruch 19, wobei das Luciferase/Luciferin-Reagens Rinderserumalbumin umfaßt, das zur Verringerung seiner Adenylatkinase-Aktivität chemisch behandelt worden ist.

21. Verfahren nach einem der vorangehenden Ansprüche, wobei die Probe mit einem Extraktionsmittel behandelt wird, welches Mikroorganismenzellen aufschließt und ihre Adenylatkinase dem ADP und den Magnesium-Ionen aussetzt.

22. Verfahren nach Anspruch 21, wobei die Zellen Pilzsporen oder somatische Zellen sind und das Extraktionsmittel ein nichtionisches Detergens umfaßt.

23. Verfahren nach Anspruch 21, wobei alle Zellen nachgewiesen werden sollen und/oder quantitativ bestimmt werden sollen und das Extraktionsmittel ein kationisches Detergens umfaßt.

24. Verfahren nach Anspruch 23, wobei das Extraktionsmittel außerdem ein oberflächenaktives Mittel umfaßt.

25. Verfahren nach Anspruch 21, wobei die Zellen Bakterienzellen sind, das durch nichtionisches Detergens freigesetzte ATP von dem ATP, das durch kationisches Detergens und oberflächenaktives Mittel freigesetzt wird, subtrahiert wird, und der Rest mit der Bakterienzellenzahl in Beziehung gesetzt wird.

26. Testkit zum Nachweis und/oder zur quantitativen Bestimmung von Mikroorganismen und/oder ihres intrazellulären Materials nach einem Verfahren nach einem der Ansprüche 1 bis 25, wobei das Kit ADP und eine Magnesium-Ionenquelle zusammen mit einem Extraktionsmittel umfaßt, um Mikroorganismenadenylatkinase diesen auszusetzen, so daß eine Umwandlung des ADPs in ATP stattfindet.

27. Testkit nach Anspruch 26, das außerdem Luciferase und Luciferin in Form eines Biolumineszenzreagenzes umfaßt, das fähig ist, Licht in Gegenwart von ATP zu emittieren.

28. Testkit nach Anspruch 27, wobei die Magnesium-Ionenquelle in Form einer Probensammlungs- oder Verdünnungslösung bereitgestellt wird.

29. Testkit nach Anspruch 28, wobei der Sammlungs- oder Verdünnungspuffer Magnesiumacetat umfaßt.

30. Testkit nach einem der Ansprüche 27 bis 29, wobei das ADP zusammen mit Detergens und/oder oberflächenaktivem Extraktionsmittel vorliegt.

31. Testkit nach einem der Ansprüche 27 bis 30, wobei das ADP, die Magnesium-Ionenquelle und das Biolumineszenzmittel in drei getrennten Behältern bereitgestellt werden.

32. Testkit nach einem der Ansprüche 27 bis 30, wobei die Mittel sämtlich als gefriergetrocknete Einzelreagenzien bereitgestellt werden.

33. Testkit nach einem der Ansprüche 26 bis 32, wobei das ADP bezüglich ATP eine Reinheit von höher 99,999 mol-% hat.

34. Testkit nach einem der Ansprüche 26 bis 33, das ein Biolumineszenzreagens mit einer Adenylatkinase-Aktivität von weniger 10⁻⁷ E/ml umfaßt.

35. Testkit nach Anspruch 33, wobei das Biolumineszenzreagens Rinderserumalbumin umfaßt, das zur Reduzierung seiner Adenylatkinase-Aktivität chemisch behandelt worden ist.

36. Reagens, das ADP mit einer Reinheit bezüglich ATP von mehr als 99,999 % umfaßt.

37. Reagens nach Anspruch 36, das außerdem einen Chelatbildner in ausreichender Menge, um zu verhindern, daß eine Adenylatkinase-Verunreinigung ADP in ATP umwandelt, umfaßt.

38. Reagens nach Anspruch 37, wobei der Chelatbildner EDTA umfaßt.

## Revendications

1. Procédé pour déterminer la présence et/ou la quantité de micro-organismes et/ou de leur matériel intracellulaire présents dans un échantillon, **caractérisé en ce que** la quantité d'adénylate-kinase dans l'échantillon est estimée en mélangeant l'échantillon avec de l'adénosine-diphosphate (ADP) et une source d'ions magnésium ajoutée en tant que réactif, en déterminant la quantité d'adénosine-triphosphate (ATP) produite par l'échantillon à partir de cette ADP, et en associant la quantité d'ATP ainsi produite à la présence/la quantité de micro-organismes et/ou de leur matériel intracellulaire.

2. Procédé selon la revendication 1, dans lequel les ions magnésium sont ajoutés à une concentration molaire suffisante pour permettre une conversion maximale d'ADP en ATP.

3. Procédé selon la revendication 1 ou 2, dans lequel la quantité d'ions magnésium ajoutés est telle qu'il y a suffisamment d'ions magnésium présents pour fournir une mole de magnésium pour une mole d'ADP de sorte que la totalité des molécules d'ADP peut être associée à au moins un ion magnésium.

4. Procédé selon l'une quelconque des revendications 1 à 3, dans lequel l'échantillon est fourni sous la forme d'une suspension ou solution aqueuse et l'estimation des micro-organismes et/ou de leur matériel intracellulaire à l'intérieur est effectuée en ajoutant l'ADP et des ions magnésium à l'échantillon sous des conditions telles que toute adénylate-kinase présente va convertir l'ADP en ATP, en incubant l'échantillon pendant une période prédéterminée pour effectuer cette conversion, en ajoutant des agents de luciférase et de luciférine, en déterminant la quantité de lumière émise par l'échantillon et en associant celle-ci à la présence et la quantité du micro-organisme et/ou du matériel intracellulaire.

5. Procédé selon l'une quelconque des revendications 1 à 4, dans lequel la quantité d'ADP avec laquelle l'échantillon est mélangé est suffisante pour fournir une concentration en ADP dans le mélange de plus de 0,005 mM.

6. Procédé selon la revendication 5, dans lequel la concentration en ADP est supérieure à 0,08 mM.

7. Procédé selon la revendication 5, dans lequel la concentration en ADP est d'environ 0,01 mM.

8. Procédé selon l'une quelconque des revendications précédentes, dans lequel la concentration d'ions magnésium dans la suspension ou solution pour la conversion d'ADP en ATP est de 1 mM ou plus.

9. Procédé selon la revendication 8, dans lequel la concentration d'ions magnésium dans la suspension ou solution est de 10 mM ou plus.

10. Procédé selon l'une quelconque des revendications précédentes, dans lequel les ions magnésium sont fournis sous la forme d'acétate de magnésium.

11. Procédé selon l'une quelconque des revendications précédentes, dans lequel les réactifs de luminescence de luciférine/luciférase sont ajoutés à l'échantillon au début de l'incubation sous forme d'un réactif unique avec l'ADP et la source d'ions magnésium.

12. Procédé selon l'une quelconque des revendications précédentes, dans lequel la source d'ions magnésium et l'ADP sont maintenues sous forme sèche ou dans des solutions séparées avant utilisation et mises ensemble ou transformées en une solution aqueuse immédiatement avant l'utilisation ou dans l'étape de conversion d'ADP.

13. Procédé selon l'une quelconque des revendications précédentes, dans lequel la source; d'ions magnésium et l'échantillon sont mélangés ensemble avant ajout de l'ADP.

14. Procédé selon la revendication 13, dans lequel l'échantillon est recueilli ou dilué dans une solution comportant la source d'ions magnésium.

15. Procédé selon l'une quelconque des revendications précédentes, dans lequel la conversion d'ADP en ATP est effectuée à un pH compris entre 5,5 et 8,5.

16. Procédé selon l'une quelconque des revendications précédentes, dans lequel l'ADP a un % molaire d'ATP inférieur à 0,001 %.

17. Procédé selon la revendication 16, dans lequel l'ADP a un % molaire d'ATP de 2 × 10⁻⁸ ou moins.

18. Procédé selon la revendication 16, dans lequel l'ADP est stockée en présence d'un agent chélatant pour empêcher que l'adénylate-kinase contaminante la convertisse en ATP de manière prématurée.

19. Procédé selon l'une quelconque des revendications précédentes, dans lequel le réactif de luciférese/luciférine a une teneur en adénylate-kinase de moins de 10⁻⁷ U/ ml.

20. Procédé selon la revendication 19, dans lequel le réactif de luciférase/luciférine comporte de l'albumine de sérum bovin qui a été chimiquement traitée pour atténuer son activité adénylate-kinase.

21. Procédé selon l'une quelconque des revendications précédentes, dans lequel l'échantillon est traité à l'aide d'un agent d'extraction qui casse des cellules de micro-organisme et expose leur adénylate-kinase à l'ADP et aux ions magnésium.

22. Procédé selon la revendication 21, dans lequel les cellules sont des spores fongiques ou des cellules somatiques et l'agent d'extraction comporte un détergent non-ionique.

23. Procédé selon la revendication 21, dans lequel toutes les cellules sont à détecter et/ou quantifier et l'agent d'extraction comporte un détergent cationique.

24. Procédé selon la revendication 23, dans lequel l'agent d'extraction comporte de plus un tensioactif.

25. Procédé selon la revendication 21, dans lequel les cellules sont des cellules bactériennes dans lesquelles l'ATP libérée par le détergent non-ionique est soustraite de l'ATP libérée par le détergent cationique et le tensioactif, et le reste est associé à des nombres de cellules bactériennes.

26. Kit d'essai pour la détection et/ou quantification de micro-organismes et/ou de leur matériel intracellulaire par un procédé selon l'une quelconque des revendications 1 à 25, ledit kit comportant de l'ADP et une source d'ions magnésium ensemble avec un agent d'extraction pour exposer l'adénylate-kinase du micro-organisme à ceux-ci de sorte qu'une conversion de l'ADP en ATP a lieu.

27. Kit d'essai selon la revendication 26, comportant de plus une luciférase et une luciférine sous la forme d'un réactif bioluminescent capable d'émettre une lumière en présence d'ATP.

28. Kit d'essai selon la revendication 27, dans lequel la source d'ions magnésium est fournie en tant que solution de dilution ou de récupération d'échantillon.

29. Kit d'essai selon la revendication 28, dans lequel le tampon de récupération ou de dilution comporte de l'acétate de magnésium.

30. Kit d'essai selon l'une quelconque des revendications 27 à 29, dans lequel l'ADP est ensemble avec l'agent d'extraction détergent et/ou tensioactif.

31. Kit d'essai selon l'une quelconque des revendications 27 à 30, dans lequel l'ADP, la source d'ions magnésium et l'agent bioluminescent sont fournis dans trois conteneurs séparés.

32. Kit d'essai selon l'une quelconque des revendications 27 à 30, dans lequel les agents sont tous fournis sous forme de réactif lyophilisé unique.

33. Kit d'essai selon l'une quelconque des revendications 26 à 32, dans lequel l'ADP est d'une pureté supérieure à 99,999 % molaire en termes d'ATP.

34. Kit d'essai selon l'une quelconque des revendications 26 à 33, comportant un réactif bioluminescent ayant une activité adénylate-kinase inférieure à 10⁻⁷ U/ ml.

35. Kit d'essai selon la revendication 33, dans lequel le réactif bioluminescent comporte de l'albumine de sérum bovin qui a été traitée chimiquement pour atténuer son activité adénylate-kinase.

36. Réactif comportant de l'ADP ayant une pureté en termes d'ATP de plus de 99,999 %.

37. Réactif selon la revendication 36, comportant de plus un agent chélatant en quantité suffisante pour empêcher qu'une adénylate-kinase contaminante ne convertisse l'ADP en ATP.

38. Réactif selon la revendication 37, dans lequel l'agent chélatant comporte EDTA.
